# EUROPEAN PATENT SPECIFICATION

(11) **EP 3 229 667 B1**
(45) Date of publication and mention of the grant of the patent: **21.10.2020**
(21) Application number: 15867423.4
(22) Date of filing: 17.09.2015
(51) Int. Cl.: A61B 5/00, A61B 5/0402, A61B 5/024, A61B 5/0408, A61B 5/0428

(54) **SENSING OF A USER'S PHYSIOLOGICAL CONTEXT USING A COMPUTING DEVICE**
ERFASSUNG EINES PHYSIOLOGISCHEN KONTEXTES EINES BENUTZERS MIT EINER COMPUTERVORRICHTUNG
DÉTECTION D'UN CONTEXTE PHYSIOLOGIQUE D'UTILISATEUR À L'AIDE D'UN DISPOSITIF INFORMATIQUE

(30) Priority: 08.12.2014 US 201414563807
(43) Date of publication of application: 18.10.2017
(73) Proprietor: Intel Corporation, Santa Clara, CA 95054 (US)
(72) Inventor: MAGESHKUMAR, Vincent S., Navi Mumbia 400709 (IN); BAXI, Amit S., Thane 400610 (IN); AHMAD, Marisa, Portland, Oregon 97221 (US); KUMAR, Arvind, Beaverton, Oregon 97006 (US)
(74) Representative: Rummler, Felix
(86) International application number: PCT/US2015/050802
(87) International publication number: WO 2016/093916

(56) References cited:
- EP-A1- 2 194 858
- EP-A1- 2 415 397
- EP-A1- 2 429 390
- WO-A1-01/75766
- WO-A1-2013/120014
- WO-A1-2014/006839
- WO-A1-2014/184868
- JP-A- 2005 122 339
- JP-A- 2008 062 033
- US-A- 6 059 819
- US-A1- 2007 027 386
- US-A1- 2010 056 880

## Description

### Field

Embodiments of the present disclosure generally relate to the field of sensor devices, and more particularly, to sensor devices for providing opportunistic measurements of user's physiological context.

### Background

Today's computing devices may provide for sensing and rendering to user some user context parameters, such as user's movements, ambient light, ambient temperature, and the like. The user context parameters may be provided by adding relevant sensors and corresponding logic to a user's computing device. However, the existing methods for provision of the user context may not include provision of user's physiological context, such as parameters related to user's state of health. Furthermore, provision of the user physiological context may consume substantial amount of user's time, and involve continuous sensor readings and corresponding data processing, which may require using substantial energy, hardware, and computing resources.

WO 2014/184868 A1 describes an apparatus pertaining to the features of the preamble of claim 1.

The present invention is defined in the independent claims. Preferred features are recited by the dependent claims,

### Brief Description of the Drawings

Embodiments will be readily understood by the following detailed description in conjunction with the accompanying drawings. To facilitate this description, like reference numerals designate like structural elements. Embodiments are illustrated by way of example and not by way of limitation in the figures of the accompanying drawings.
FIG. 1 is a block diagram illustrating an apparatus for opportunistic measurements of user's physiological context, incorporated with the teachings of the present disclosure, in accordance with some embodiments.
FIG. 2 is a schematic diagram illustrating an example apparatus for opportunistic measurements of user's physiological context, in accordance with some embodiments.
FIG. 3 is a schematic diagram illustrating an example implementation of a sensor arrangement on a work surface of a computing device, to enable opportunistic measurements of user's physiological context, in accordance with some embodiments.
FIG. 4 illustrates example shapes of electrically conductive patterns that may be disposed on a work surface of a computing device, in accordance with some embodiments.
FIG. 5 illustrates examples of disposition of sensors on work surfaces of computing devices, to enable measurements of a user's physiological context, in accordance with some embodiments.
FIG. 6 is a schematic diagram illustrating an electrically conductive pattern assembly disposed on a work surface of a computing device (e.g., keyboard) and configured to expand a sensing surface of a capacitive electrode for opportunistic ECG measurements, in accordance with the present invention.
FIGS. 7-8 illustrate different views of an example apparatus for opportunistic measurements of user's physiological context, in accordance with some embodiments.
FIG. 9 illustrates an example circuit board implementing the circuitry enabling opportunistic measurements of the user's physiological context, in accordance with some embodiments.
FIG. 10 is a process flow diagram for assembling an apparatus for opportunistic measurements of user's physiological context, in accordance with some embodiments.
FIG. 11 illustrates an example computing device suitable for use with various components of FIG. 1, such as apparatus for opportunistic measurements of user's physiological context of FIG. 1, in accordance with some embodiments.

### Detailed Description

Embodiments of the present disclosure include techniques and configurations for opportunistic measurements of user's physiological context. Opportunistic measurements may include measurements of user's physiological context, e.g., during user's interaction with the apparatus, when at least portions of user's limbs (e.g., hands, palms, and/or wrists) are disposed on the work surface of the apparatus to interact with the apparatus. In accordance with embodiments, the apparatus may comprise a work surface that includes one or more electrodes disposed on the work surface to directly or indirectly (e.g., when the electrodes are covered by, or placed behind, an enclosure of the apparatus) contact with portions of user's limbs (hands, palms, or wrists), when the user's portions of limbs are disposed on the work surface to interact with the apparatus, to obtain one or more parameters of user's physiological context. During the interaction, the portions of user's limbs may maintain direct or indirect contact with the electrodes, allowing for measurements of the user's physiological context. The apparatus may further include circuitry coupled with the electrodes to detect direct or indirect or indirect contact between the user's portions of limbs and the electrodes and on detection, collect the parameters of the user's physiological context while the direct or indirect contact is maintained.

The example embodiments describe contact between different portions of user's limbs, such as hands, palms, or wrists, and the sensors (e.g. electrodes) of the apparatus. Different other embodiments may be contemplated, wherein other portions of user's limbs may interact with the apparatus, allowing for measurements of the user's context, such as elbows, forearms, and the like.

In the following detailed description, reference is made to the accompanying drawings that form a part hereof, wherein like numerals designate like parts throughout, and in which are shown by way of illustration embodiments in which the subject matter of the present disclosure may be practiced. It is to be understood that other embodiments may be utilized and structural or logical changes may be made without departing from the scope of the present disclosure. Therefore, the following detailed description is not to be taken in a limiting sense, and the scope of embodiments is defined by the appended claims and their equivalents.

For the purposes of the present disclosure, the phrase "A and/or B" means (A), (B), or (A and B). For the purposes of the present disclosure, the phrase "A, B, and/or C" means (A), (B), (C), (A and B), (A and C), (B and C), or (A, B, and C).

The description may use perspective-based descriptions such as top/bottom, in/out, over/under, and the like. Such descriptions are merely used to facilitate the discussion and are not intended to restrict the application of embodiments described herein to any particular orientation.

The description may use the phrases "in an embodiment," or "in embodiments," which may each refer to one or more of the same or different embodiments. Furthermore, the terms "comprising," "including," "having," and the like, as used with respect to embodiments of the present disclosure, are synonymous.

The term "coupled with," along with its derivatives, may be used herein. "Coupled" may mean one or more of the following. "Coupled" may mean that two or more elements are in direct physical, electrical, or optical contact. However, "coupled" may also mean that two or more elements indirectly contact each other, but yet still cooperate or interact with each other, and may mean that one or more other elements are coupled or connected between the elements that are said to be coupled with each other. The term "directly coupled" may mean that two or more elements are in direct or indirect contact.

FIG. 1 is a block diagram illustrating an apparatus 100 for opportunistic measurements of user's physiological context, incorporated with the teachings of the present disclosure, in accordance with some embodiments. The apparatus 100 may comprise a work surface 102, e.g., a portion of a keyboard or other part of a computing device 104. The work surface 102 may include one or more sensors (e.g., electrodes) 110, 112, 114, 116 and other sensors 118, 120 disposed on the work surface to directly or indirectly contact with portions of user's limbs, e.g., hands, palms, or wrists 106, when the user's hands, palms or wrists 106 are disposed on the work surface 102 to interact with the apparatus 100, to obtain one or more parameters of user's physiological context. The electrodes 110, 112, 114, 116 and sensors 118 and 120 may provide readings related to various user body functions as discussed below in greater detail. For example, electrodes may 110, 112, 114, 116 may be configured to measure electrocardiogram (ECG) bio-potentials from the user's hands, and sensors 118 and 120 may comprise optical sensors to provide photoplethysmographic (PPG) measurements and skin temperature sensors to measure body temperature. Electrodes 110, 112, 114, and 116 may form an electrically conductive pattern 160 on the work surface 102, and sensors 118 and 120 may form a sensor array 162 on the work surface 102, as will be described below in greater detail. One skilled in the art will appreciate that a number of electrodes and sensors illustrated in FIG. 1 and types of sensors are provided for illustration purposes only and are not limiting this disclosure. Different types of sensors providing readings of user's physiological context may be disposed on (e.g., embedded in) the work surface 102 as will be described below.

The apparatus 100 may further comprise electronic circuitry 124 coupled with the electrodes 110, 112, 114, 116, and sensors 118 and 120, to detect direct or indirect or indirect contact between the user's hands, palms, or wrists 106 and the electrodes 110, 112, 114, 116 and/or sensors 118 or 118 and on detection, collect parameters of the user's physiological context while the direct or indirect contact is maintained, thus enabling opportunistic measurements of the user's physiological context. The circuitry 124 may include ECG module 126 to provide opportunistic sensing and pre-processing of ECG measurements, PPG module 128 to provide opportunistic sensing and pre-processing of PPG measurements, temperature module 130 to provide opportunistic sensing and pre-processing of body skin temperature, and detection module 132 to detect direct or indirect or indirect contact between the user's hands, palms, or wrists 106 and at least some of the electrodes 110, 112, 114, 116 or sensors 118, 120, to initiate the opportunistic measurements while the direct or indirect contact is maintained.

The apparatus 100 may further include a processing unit 140 configured to process the readings provided by the electrodes and sensors 110-120 and collected by the circuitry 124. For example, the processing unit 140 may include a respiration rate determination module 142 to determine user's respiration rate based, e.g., on readings provided by ECG module 126. The processing unit 140 may further include a blood pressure determination module 144 to provide estimates of the user's blood pressure based on readings provided by ECG module 126 and PPG module 128. The provision of blood pressure and respiration rate parameters may be done empirically or heuristically, e.g., using machine-learning algorithms, and is not a subject of the present disclosure.

In some embodiments, the processing unit 140 may include a processor 146 configured to process the readings (signals) provided by the sensors circuitry 124, and memory 148 having instructions that, when executed on the processor 146, may cause the processor 146 to perform signal processing as described above. The processing unit 140 may include other components 150 necessary for the functioning of the apparatus 100. For example, the processing unit 140 may be coupled with one or more interfaces (not shown) to communicate the user's physiological context measurements over one or more wired or wireless network(s) and/or with any other suitable device, such as external computing device 154.

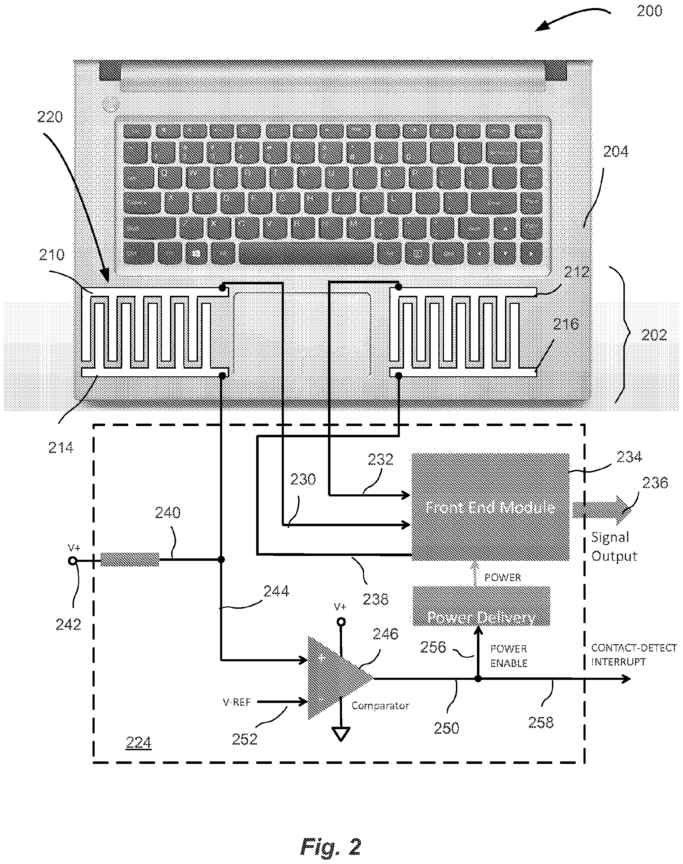

FIG. 2 is a schematic diagram illustrating an example apparatus 200 for opportunistic measurements of user's physiological context, in accordance with some embodiments. The apparatus 200 may include one or more components of the apparatus 100 of FIG. 1 described above.

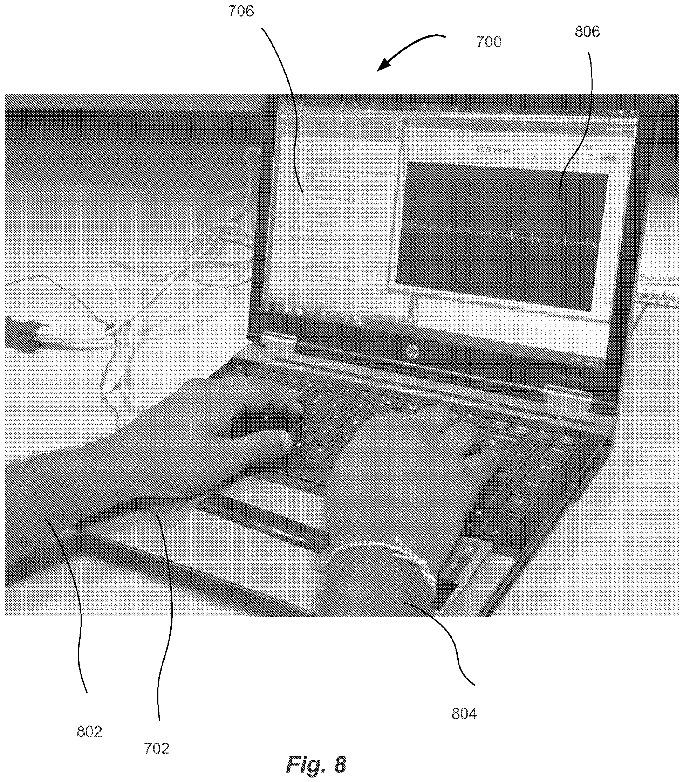

As described in reference to FIG. 1, apparatus 200 may include a work surface 202, in this example, a surface of a computing device, such as computing device keyboard 204, which may come in direct or indirect contact with user's hands when the user operates the keyboard 204. The apparatus 200 may include electrodes 210, 212, 214, and 216 that may form an electrically conductive pattern 220 laid on the work surface 202 of the keyboard 204. In the example of FIG. 1, the electrically conductive pattern 220 may comprise a comb pattern. The electrodes 210, 212, 214, and 216 forming electrically conductive pattern 220 may be made of, for example, a metallic film, or may be non-metallic, e.g., may be made of conductive screen, printed conductive ink, conductive fabric or conductive elastomer. Electrodes 210 and 212 may be used to sense ECG bio-potentials from left and right hands (palms or wrists) of the user (see FIG. 8). Electrode 216 may be a common electrode, and electrode 214 (hereinafter contact detect electrode) may serve to detect direct or indirect contact between user's hands, palms or wrists and the electrically conductive pattern 220.

The signals 230 and 232 from the electrodes 210 and 212 may be fed to circuitry 224 (such as circuitry 124 of FIG. 1). Circuitry 224 may include a front end sensor module 234 to receive and pre-process readings (e.g., signals 230 and 232) during the direct or indirect contact with the user's hands, palms or wrists. To that end, the front end sensor module 234 may include an amplifier, an analog-to-digital converter (ADC) and a controller to operate the circuitry 224. In the illustrated example of ECG readings collection, the front-end sensor module 234 may derive a differential ECG signal from the input signals 230 and 232, and digitize the signal to produce an output digital ECG signal 236. A signal 238 from the common electrode 216 may be used as a reference signal and to reduce the common-mode noise in ECG readings provided by 230 and 232.

ECG may be measured when portions of user's limbs (hands, palms, fingers, or wrists) of one or both hands (for reliable measurements) make contact with the work surface 202. Accordingly, it may be desirable to detect an instance when the ECG sensing surfaces (e.g., electrically conductive pattern 220) may be in direct touch (contact) with user's hands, palms, or wrists so that the front-end sensor module 234 may be powered on when ECG may be reliably sensed by the electrically conductive pattern 220. Because the ECG is to be sensed opportunistically, rather than keeping the front end module 234 always powered on, a direct or indirect contact detection technique may be used to detect contact of both hands, palms, or wrists with the work surface 202 (and accordingly with electrically conductive pattern 220) of the keyboard 204. The technique described below may conserve system power and eliminate the need for the system processor (e.g., 146 of FIG. 1) to continuously acquire the differential signal and continuously analyze the differential signal to detect valid ECG signals, as provided by conventional systems.

The direct or indirect contact detect technique may be implemented by a combination of contact detect electrode 214 and the common electrode 216. The contact detect electrode 214 may be always maintained at a determined (e.g., high potential) via a high impedance pull-up 240 connected to the positive supply rail 242. The same voltage signal 244 may be brought to the positive input of comparator 246. The output signal 250 of the comparator 246 may be normally "high" since its voltage is greater than the voltage V-REF of signal 252 at negative input of the comparator 246. When one hand, palm or wrist of the user (not shown) touches electrodes 210, 214 and the other hand, palm, or wrist touches electrodes 212, 216, the voltage signal 244 at positive input of comparator 246 may drop below V-REF, causing the output signal 250 of comparator 246 to switch from "high" to "low." This drop in comparator 246's output voltage signal 250 may be used to detect contact of both hands (palms, wrists) on the work surface 202 and turn on power to the front end sensor module 234 using power enable signal 256, via a power delivery network circuit 254. At the same time, signal 250 may be provided as a notification (contact detect interrupt 258) to the system processor (e.g., 146), so that it may begin acquiring ECG data (e.g., output signal 236) from the electrodes 210 and 212.

In some embodiments, the direct or indirect contact detect technique may be implemented, for example, by sensing pressure at touch surfaces on the work surface (e.g., keyboard), using pressure sensors such as strain gauge or force sensitive resistors.

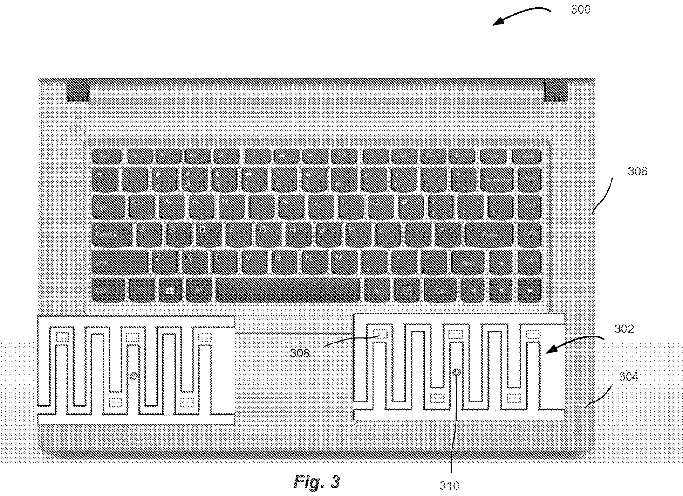

FIG. 3 is a schematic diagram illustrating an example implementation of a sensor arrangement 300 on a work surface of a computing device, to enable opportunistic measurements of user's physiological context, in accordance with some embodiments.

The electrically conductive pattern 302 is shown as disposed on a work surface 304 of a computing device 306. As described in reference to FIG. 2, the sensor arrangement 300 may include comb-patterned electrodes comprising the conductive pattern 302 used to measure ECG. In addition or in the alternative, the sensor arrangement 300 may include an array of optical sensors 308 to provide PPG measurements as briefly described in reference to FIG. 1. The optical sensors 308 may comprise a combination of photodetectors and light-emitting diodes (LED) configured to detect a flow of blood, e.g., to user's fingers or palms placed around the work surface 202, from which data blood pressure of the user may be derived (e.g., in combination with ECG readings as described in reference to FIG. 1). The sensor arrangement 300 may further include one or more temperature sensors 310 disposed on the work surface 302 as shown to measure user's body temperature. The temperature sensors may either be contact type (e.g. thermistors or thermocouples) or non-contact type (e.g. infra-red radiation sensors). Accordingly, direct contact with a work surface (and sensors) may not be needed for measuring user's body temperature.

As described in reference to FIGS. 2-3, the conductive electrodes disposed on a work surface of a computing device may comprise an electrically conductive pattern. FIGS. 2-3 illustrated electrically conductive patterns in a shape of a comb. However, many different shapes of electrically conductive patterns may be used in opportunistic measurements of the user's physiological context as described herein.

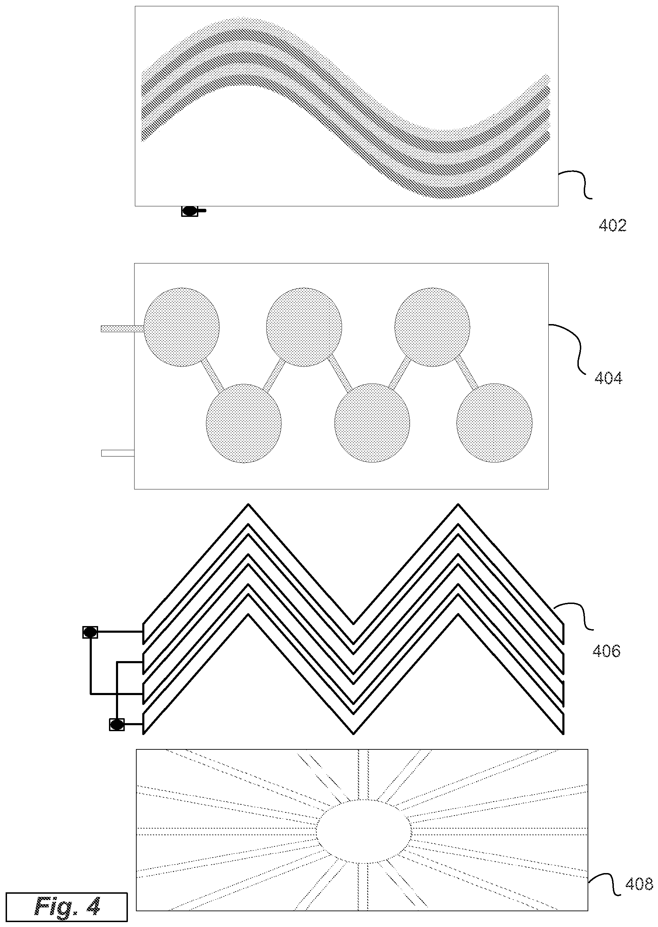

FIG. 4 illustrates example shapes of electrically conductive patterns that may be disposed on a work surface of a computing device, in accordance with some embodiments. As shown, the electrically conductive pattern that may be disposed on a work surface of a computing device may comprise a wave pattern 402, garland pattern 404, zigzag pattern 406, or a sunbeam pattern 408. The illustrated shapes of electrically conductive patterns do not limit this disclosure; one skilled in the art will appreciated that a variety of shapes of electrically conductive patterns may be disposed on a work surface of a computing device as suitable for measuring the user's physiological context.

In order to allow for seamless opportunistic sensing of user's physiological context, the user may need to have access to the sensors providing measurements of user's physiological context in natural positions and during regular user activities, such as during operation of a computing device. Accordingly, in addition or in the alternative to the placement of sensors on a work surface of a computing device described in reference to FIGS. 2-3, the sensors may be placed in various portions of a computing device, with which the user may come in direct or indirect contact, depending on a type of a device. For example, the sensors may be placed in various parts of a casing of a computing device.

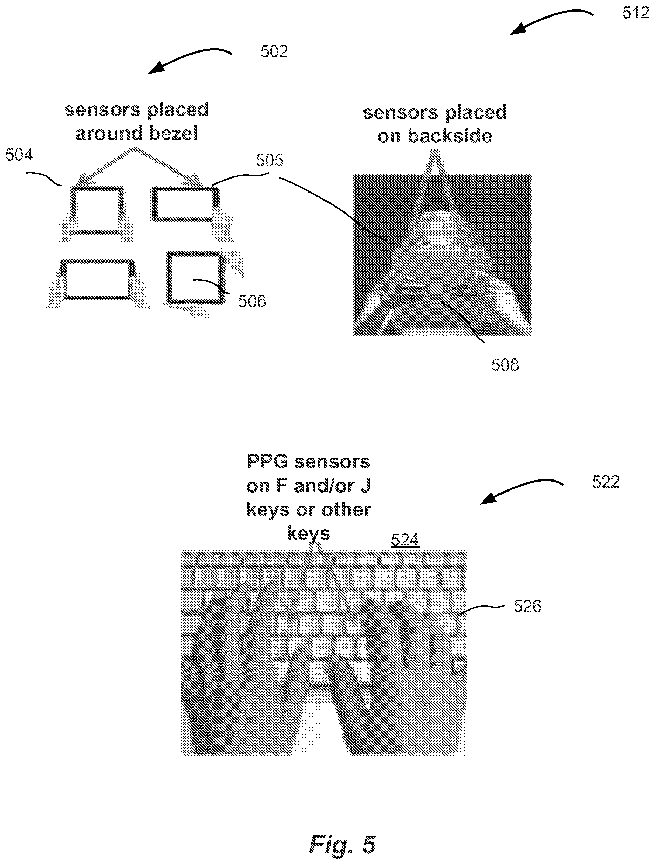

FIG. 5 illustrates examples of disposition of sensors on work surfaces of computing devices, to enable measurements of a user's physiological context, in accordance with some embodiments. View 502 illustrates the placement of the sensors around a bezel 504 of a casing 505 of a tablet computing device or a smart phone 506. View 512 illustrates the placement of the sensors around a back side 508 of the casing 505 of a tablet computing device or a smart phone (e.g., 506). View 522 illustrates the placement of the sensors on a keyboard 526 of a computing device, such as a laptop, tablet (if equipped with a keyboard), or desktop computer. As shown, the sensors may be disposed on particular keys 524 of the keyboard 526. Accordingly, the casing with a work surface suitable for placing the sensors for measurements of a user's physiological context may include at least a portion of a keyboard of a computing device, a bezel of the computing device, or a back side of the computing device. In summary, a computing device, on which the sensors for opportunistic measurements of the user's physiological context may be placed, may include a laptop computer, a desktop computer, a tablet computer, a smart phone, or any other mobile or stationary computing device.

The electrically conductive patterns described in reference to FIGS. 2-3 and 5 may be used to provide ECG readings, when placed on a work surface of a computing device. The quality of ECG signals provided by electrically conductive patterns may depend on the quality of contact of the user's hand, palms, or wrists with the electrodes. If the user's hands, palms, or wrists are dry, the signal quality may deteriorate. It may be beneficial to use capacitive electrodes for ECG measurements instead of metallic electrodes (e.g., instead of electrically conductive patterns described above) to improve ECG signal quality in opportunistic measurements of user's physiological context.

Capacitive electrodes sense electric potential between two plates (surfaces) of the
capacitor. The capacitive electrodes may have a relatively small sensing surface area (typically about 10 sq. mm). The sensing surface of such capacitive electrodes may be expanded by increasing the plate area (and hence the sensing surface) of the capacitive electrode. The sensing surface expansion may be accomplished by electrically connecting the sensing surface of the capacitor to a much larger conductive surface, for example, the electrically conductive pattern that may be mounted on the work surface of a casing of a computing device as described above.

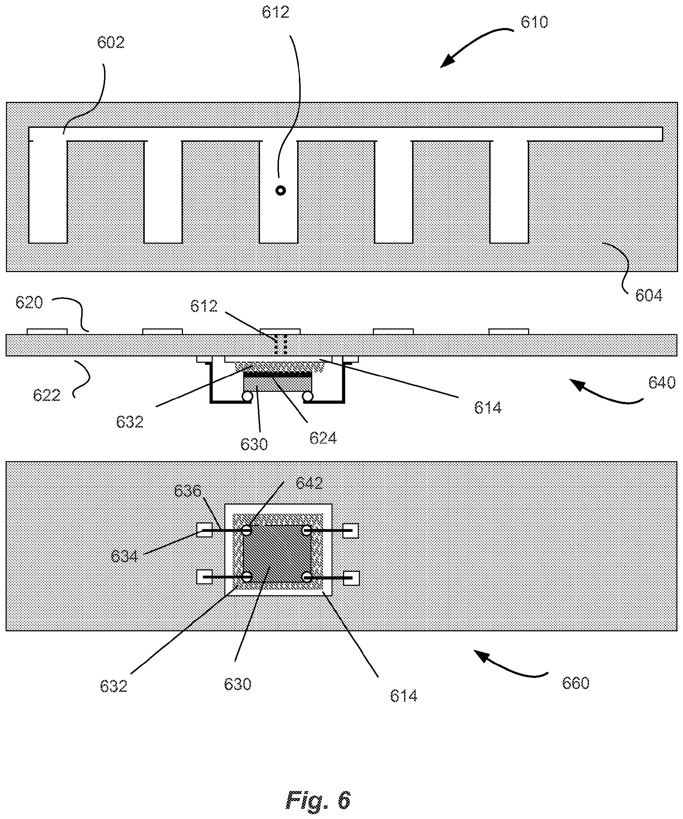

FIG. 6 is a schematic diagram illustrating an electrically conductive pattern assembly disposed on a work surface of a computing device (e.g., keyboard) and configured to expand a sensing surface of a capacitive electrode for opportunistic ECG measurements, in accordance with the present invention. More specifically, FIG. 6 illustrates a top view 610, a side view 640, and a bottom view 660 of the electrically conductive pattern assembly.

As described above, an electrically conductive electrode pattern 602 (e.g., large sensing surface) may be created on a substrate 604, e.g., by film deposition or etching. The substrate 604 may comprise a glass epoxy substrate (e.g., FR4) of a printed circuit board (PCB). Alternatively, the substrate 604 may comprise a casing of a computing device (e.g., a casing of a keyboard described in reference to FIG. 2). The casing may be made of a plastic material. An electrical connection 612 may be provided from the electrode pattern 602 from a top surface 620 of the substrate 604 to a conductive plane 614 of a capacitive electrode 630 disposed on a bottom surface 622 of the substrate 602, to facilitate electrical connection with a sensing surface 624 of the capacitive electrode 630.

For a robust electrical connection, a flexible conductive washer 632 may be used between the conductive plane 614 and sensing surface 624. The conductive washer 632 may be made, for example, from a conductive textile or elastomer. The capacitive electrode 630 may be mounted on the bottom surface 622 of the substrate 604 using, for example, conductive solderable pads 634, mounting studs 642, and metallic pins 636. Other variants of the assembly of FIG. 6 may be possible to achieve the similar functionality.

The embodiments described in reference to FIGS. 1-6 may provide the following advantages. Providing capacitive ECG on a work surface of a computing device may result in an ECG signal of desired quality, even when a user may have dry hands, palms, or wrists. In other words, the described embodiments may not require user's hands, palms, or wrists to be moist in order to conduct opportunistic measurements of user's physiological context. Further, measurements of the user's physiological context may be conducted when the pressure of user's hands, palms, or wrists on the working surface may be below a certain level. Namely, the user may not need to apply any additional pressure to the work surface in order of the measurements of the user's physiological context to occur. Further, due to opportunistic character of measurements, the embodiments described in reference to FIGS. 1-6 may provide for reduced power consumption, compared to conventional techniques, when the sensors and corresponding processing units may be always powered on.

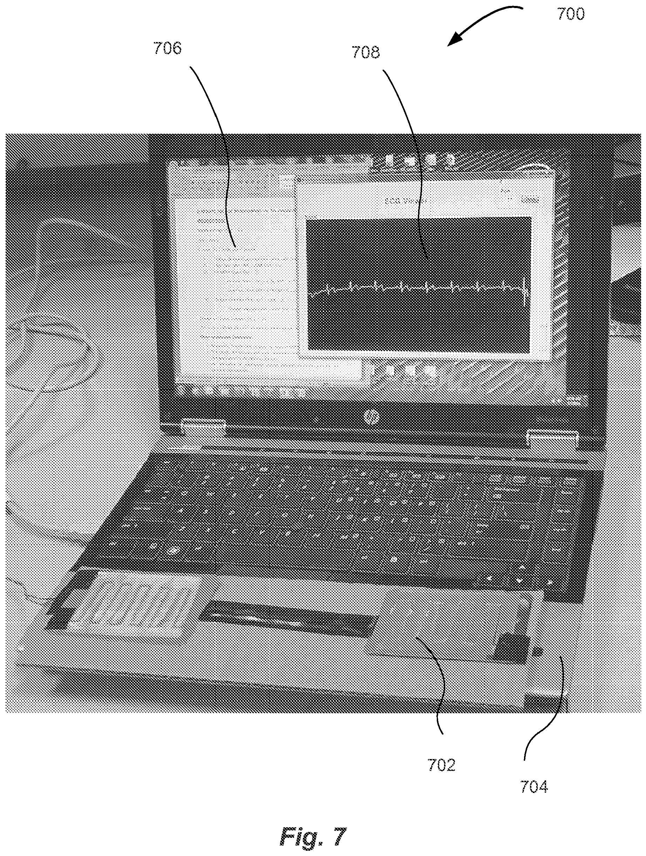

The described embodiments may enable several applications, such as in cardiac health monitoring, arrhythmia detection, normal or abnormal ECG classification, cardiac health trends, biometric authentication, and the like. ECG measurements may also be used for other applications such as heart rate monitoring, emotional monitoring, stress detection, and the like. As illustrated in FIGS. 7-8, the described embodiments may be deployed in existing keyboards and docking stations of computing devices.

FIGS. 7-8 illustrate different views of an example apparatus for opportunistic measurements of user's physiological context, in accordance with some embodiments. FIG. 7 illustrates a laptop computer 700 with a mock up electrically conductive electrode pattern 702 disposed on a work surface (portion of a keyboard) 704. The circuitry 124 and processing module 140 described in reference to FIG. 1, although not visible in FIG. 7, provide for displaying on a computer screen 706 the ECG results 708 measured by the electrically conductive pattern 702 when the user's hands were in contact with electrode pattern 702.

FIG. 8 illustrates the laptop computer 700 wherein the electrically conductive electrode pattern 702 is shown during the direct contact with user's wrists 802, 804, providing ECG measurements 806 on the computer screen 706.

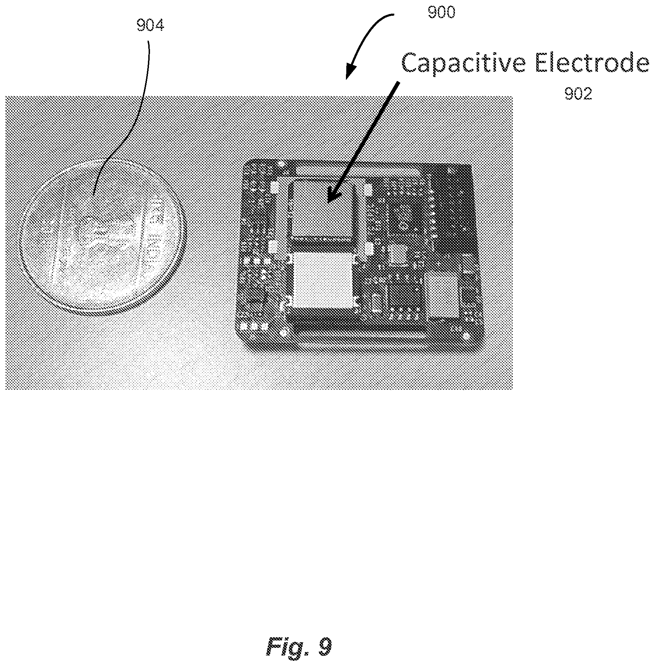

FIG. 9 illustrates an example circuit board 900 implementing the circuitry enabling opportunistic measurements of the user's physiological context, in accordance with some embodiments. The circuit board 900 may include the components of circuitry 124 and 224 described in reference to FIGS. 1 and 2. The circuit board 900 may be applied to the embodiments described in reference to FIG. 6. The capacitive electrode 902 (similar to 630) is shown as coupled with the circuit board 900 to implement the embodiments of FIG. 6. The size of the circuit board 900 and capacitive electrode 902 may be appreciated if compared to a size of the coin 904 (about 20 mm in diameter) placed in proximity to the circuit board 900.

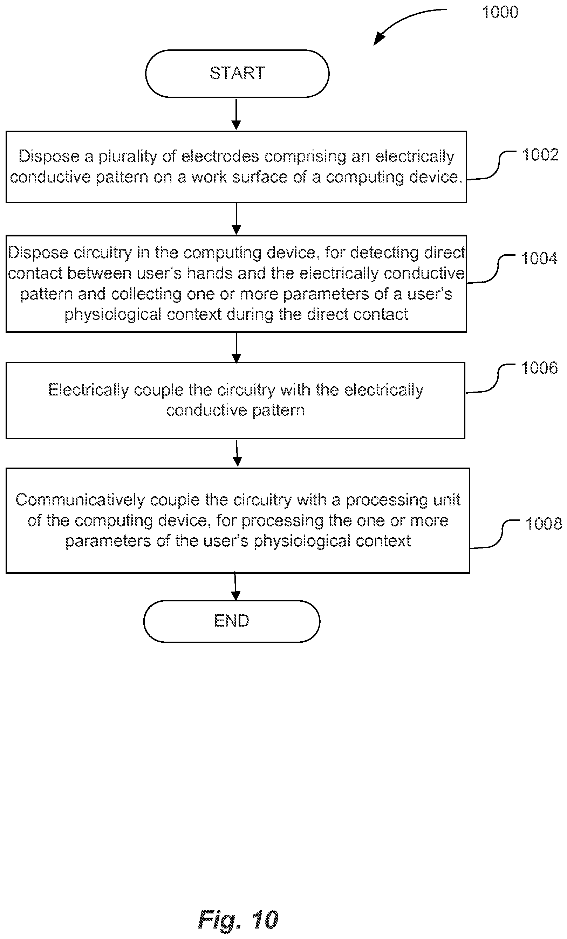

FIG. 10 is a process flow diagram for assembling an apparatus for opportunistic measurements of user's physiological context, in accordance with some embodiments. The process 1000 may comport with some of the apparatus embodiments described in reference to FIGS. 1-9. In alternate embodiments, the process 1000 may be practiced with more or less operations, or different order of the operations.

The process 1000 may begin at block 1002 and include disposing a plurality of electrodes comprising an electrically conductive pattern on a work surface of a computing device. Disposing an electrically conductive pattern may include etching or depositing the electrically conductive pattern on a substrate comprising the work surface. In some embodiments, disposing the electrically conductive pattern on the work surface may include printing the electrically conductive pattern in a form of a sticker and affixing the sticker to the work surface.

At block 1004, the process 1000 may include disposing circuitry in the computing device, for detecting direct or indirect contact between portions of user's limbs (e.g., hands, palms, or wrists) and the electrically conductive pattern and collecting one or more parameters of a user's physiological context during the direct or indirect contact.

At block 1006, the process 1000 may include electrically coupling the circuitry with the electrically conductive pattern.

At block 1008, the process 1000 may include communicatively coupling the circuitry with a processing unit of the computing device, for processing the one or more parameters of the user's physiological context.

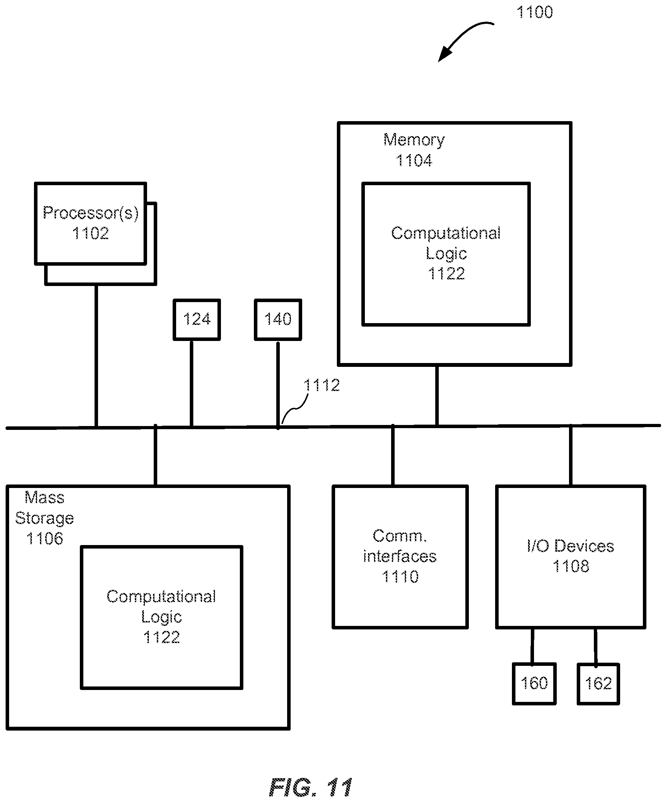

FIG. 11 illustrates an example computing device 1100 having various components of FIG. 1, such as apparatus 100 for opportunistic measurements of user's physiological context of FIG. 1, in accordance with some embodiments. In some embodiments, example computing device 1100 may include various components of apparatus 100, e.g., the circuitry 124 and/or processing unit 140 described in reference to FIG. 1. In some embodiments, various components of the example computing device 1100 may be used to interface with the external device 154. As shown, computing device 1100 may include one or more processors or processor cores 1102 and system memory 1104. For the purpose of this application, including the claims, the terms "processor" and "processor cores" may be considered synonymous, unless the context clearly requires otherwise. The processor 1102 may include any type of processors, such as a central processing unit (CPU), a microprocessor, and the like. The processor 1102 may be implemented as an integrated circuit having multi-cores, e.g., a multi-core microprocessor. The computing device 1100 may include mass storage devices 1106 (such as solid state drives, volatile memory (e.g., dynamic random-access memory (DRAM), and so forth).

In general, system memory 1104 and/or mass storage devices 1106 may be temporal and/or persistent storage of any type, including, but not limited to, volatile and non-volatile memory, optical, magnetic, and/or solid state mass storage, and so forth. Volatile memory may include, but is not limited to, static and/or dynamic random-access memory. Non-volatile memory may include, but is not limited to, electrically erasable programmable read-only memory, phase change memory, resistive memory, and so forth.

The computing device 1100 may further include input/output (I/O) devices 1108 (such as a display, keyboard, touch sensitive screen, image capture device, and so forth) and communication interfaces 1110 (such as network interface cards, modems, infrared receivers, radio receivers (e.g., Near Field Communication (NFC), Bluetooth, WiFi, 4G/5G LTE), and so forth).

The communication interfaces 1110 may include communication chips (not shown) that may be configured to operate the device 1100 in accordance with a Global System for Mobile Communication (GSM), General Packet Radio Service (GPRS), Universal Mobile Telecommunications System (UMTS), High Speed Packet Access (HSPA), Evolved HSPA (E-HSPA), or Long-Term Evolution (LTE) network. The communication chips may also be configured to operate in accordance with Enhanced Data for GSM Evolution (EDGE), GSM EDGE Radio Access Network (GERAN), Universal Terrestrial Radio Access Network (UTRAN), or Evolved UTRAN (E-UTRAN). The communication chips may be configured to operate in accordance with Code Division Multiple Access (CDMA), Time Division Multiple Access (TDMA), Digital Enhanced Cordless Telecommunications (DECT), Evolution-Data Optimized (EV-DO), derivatives thereof, as well as any other wireless protocols that are designated as 3G, 4G, 5G, and beyond. The communication interfaces 1110 may operate in accordance with other wireless protocols in other embodiments.

The above-described computing device 1100 elements may be coupled to each other via system bus 1112, which may represent one or more buses. In the case of multiple buses, they may be bridged by one or more bus bridges (not shown). Each of these elements may perform its conventional functions known in the art. In particular, system memory 1104 and mass storage devices 1106 may be employed to store a working copy and a permanent copy of the programming instructions implementing the operations associated with the apparatus 100, such as modules 142 and 144 described in reference to the processing unit 140 of FIG. 1. The various elements may be implemented by assembler instructions supported by processor(s) 1102 or high-level languages that may be compiled into such instructions.

The permanent copy of the programming instructions may be placed into permanent storage devices 1106 in the factory, or in the field, through, for example, a distribution medium (not shown), such as a compact disc (CD), or through communication interface 1110 (from a distribution server (not shown)). That is, one or more distribution media having an implementation of the agent program may be employed to distribute the agent and to program various computing devices.

The number, capability, and/or capacity of the elements 1108, 1110, 1112 may vary, depending on whether computing device 1100 is used as a stationary computing device, such as a set-top box or desktop computer, or a mobile computing device, such as a tablet computing device, laptop computer, game console, or smartphone. Their constitutions are otherwise known, and accordingly will not be further described.

At least one of processors 1102 may be packaged together with computational logic 1122 configured to practice aspects of embodiments described in reference to FIGS. 1-10. For one embodiment, at least one of processors 1102 may be packaged together with memory having computational logic 1122 to form a System in Package (SiP) or a System on Chip (SoC). For at least one embodiment, the SoC may be utilized in, e.g., but not limited to, a computing device such as a laptop, desktop, computing tablet or smartphone.

In embodiments, the computing device 1100 may include at least some of the components of the apparatus 100 as described above. In some embodiments, the apparatus 100 may include sensor module (electrically conductive pattern) 160, sensor array 162 (e.g., disposed on a keyboard of the computing device 1100). Circuitry 124, and processing unit 140 and may be communicatively coupled with the computing device 1100 as shown in FIG. 11 and described herein.

In various implementations, the computing device 1100 may comprise a laptop, a netbook, a notebook, an ultrabook, a smartphone, a tablet, a personal digital assistant (PDA), an ultra mobile PC, a mobile phone, a laptop, a desktop, or any other mobile computing device. In further implementations, the computing device 1100 may be any other electronic device that processes data.

Various operations are described as multiple discrete operations in turn, in a manner that is most helpful in understanding the claimed subject matter. However, the order of description should not be construed as to imply that these operations are necessarily order dependent. Embodiments of the present disclosure may be implemented into a system using any suitable hardware and/or software to configure as desired.

Although certain embodiments have been illustrated and described herein for purposes of description, a wide variety of alternate and/or equivalent embodiments or implementations calculated to achieve the same purposes may be substituted for the embodiments shown and described without departing from the scope of the present disclosure. This application is intended to cover any adaptations or variations of the embodiments discussed herein. Therefore, it is manifestly intended that embodiments described herein be limited only by the claims.

## Claims

1. An apparatus (100, 200, 300) for opportunistic measurements of user's context, said measurements including ECG measurements, said apparatus being a computing device comprising:
at least one work surface (102, 202, 304) that includes one or more electrodes (110, 112, 114, 116; 210, 212, 214, 216) disposed on the work surface to directly contact with user's hands, palms, or wrists, when the user's hands, palms, or wrists are disposed on the work surface to interact with the apparatus, to obtain one or more parameters of user's physiological context; and
circuitry (124, 224) coupled with the electrodes to detect direct contact between the user's hands, palms, or wrists and the electrodes and on detection, collect the one or more parameters of the user's physiological context while the direct contact is maintained,
wherein the one or more electrodes form an electrically conductive pattern (160, 220, 302) on the work surface, **characterized in that** the electrically conductive pattern is electrically coupled with a sensing surface of a capacitive ECG measurement electrode (630) disposed inside the work surface or on a back side of the work surface, wherein the size of the electrically conductive pattern is greater than the size of the sensing surface, to expand the sensing surface of the capacitive electrode.

2. The apparatus of claim 1, wherein the circuitry comprises:
at least one of the one or more electrodes to detect direct contact, with a determined electric potential; and
a comparator coupled with the at least one electrode to detect a change in the determined electric potential, wherein the change is caused by the direct contact of the at least one electrode with the user's hands, palms, or wrists, wherein the comparator is to provide output that enables powering on of the electrically conductive pattern as a result of the detection of the change in the determined electric potential, wherein the circuitry further comprises a front end sensor module to receive and pre-process readings provided by the electrically conductive pattern during the direct contact with the user's hands, palms, or wrists, wherein the comparator output further enables powering on the front end sensor module and the electrically conductive pattern.

3. The apparatus of claim 2, wherein the electrically conductive pattern comprises a selected one of: a comb pattern, a zigzag pattern, a wave pattern, or a garland pattern.

4. The apparatus of claim 2, wherein the work surface comprises a substrate, wherein the electrically conductive pattern is disposed on an outer side of the substrate, and wherein the capacitive electrode is disposed on an inner side of the substrate, wherein the electrically conductive pattern is disposed on the substrate by film deposition, etching, or affixing an electrically conductive sticker comprising the pattern to the substrate.

5. The apparatus of claim 2, wherein the electrically conductive pattern comprises at least two electrocardiogram (ECG) electrodes.

6. The apparatus of claim 2, wherein the electrically conductive pattern is coupled with one or more of: a temperature sensor to provide user's body temperature, or an optical sensor to provide a photoplethysmogram (PPG) of the user wherein the circuitry is to provide the parameters of the user's physiological context to a processing unit associated with the apparatus for further processing, wherein the user's physiological context comprises at least some of: electrocardiographic data, photoplethysmographic data, blood pressure, temperature, and respiration.

7. The apparatus of claim 1, wherein the apparatus is a laptop computer or a desktop computer, wherein the work surface comprises a part of a keyboard of the laptop computer or the desktop computer.

8. The apparatus of any of claims 1 to 7, wherein the apparatus is a tablet computer or a smart phone, wherein the work surface comprises a selected one of a bezel of the tablet computer or a back side of the tablet computer or the smart phone.

9. The apparatus of claim 1, comprising a casing, having the at least one work surface.

10. The apparatus of claim 9, wherein the casing comprises at least a portion of a keyboard of the apparatus, a bezel of the apparatus, or a back side of the apparatus.

11. The apparatus of claim 10, wherein the apparatus comprises one of: a laptop computer, a desktop computer, a tablet computer, or a smart phone.

12. A method (1000) of assembling an apparatus for opportunistic measurements of user's context, said measurements comprising ECG measurements, said method comprising:
disposing (1002) a plurality of electrodes comprising an electrically conductive pattern on a work surface of a computing device;
disposing (1004) circuitry in the computing device, for detecting direct contact between user's hands or wrists and the electrically conductive pattern and collecting one or more parameters of a user's physiological context during the direct contact; and
electrically coupling (1006) the circuitry with the electrically conductive pattern,
**characterized in that**
the electrically conductive pattern is electrically coupled with a sensing surface of a capacitive ECG measurement electrode disposed inside the work surface or on a back side of the work surface, wherein the size of the electrically conductive pattern is greater than the size of the sensing surface, to expand the sensing surface of the capacitive electrode.

13. The method of claim 12, wherein disposing an electrically conductive pattern comprises etching, depositing the electrically conductive pattern on a substrate comprising the work surface, or affixing an electrically conductive sticker comprising the pattern to the substrate.

14. The method of claim 12 or 13, further comprising: communicatively coupling the circuitry with a processing unit of the computing device, for processing the one or more parameters of the user's physiological context.

## Patentansprüche

1. Einrichtung (100, 200, 300) für opportunistische Messungen des Kontextes eines Benutzers, wobei die Messungen EKG-Messungen beinhalten und die Einrichtung eine Computervorrichtung ist, umfassend:
wenigstens eine Arbeitsfläche (102, 202, 304), die eine oder mehrere Elektroden (110, 112, 114, 116; 210, 212, 214, 216) aufweist, die auf der Arbeitsfläche angeordnet sind, um in direkten Kontakt mit den Händen, Handflächen oder Handgelenken des Benutzers zu treten, wenn die Hände, Handflächen oder Handgelenke des Benutzers auf der Arbeitsfläche angeordnet sind, um mit der Einrichtung zu interagieren, um einen oder mehrere Parameter des physiologischen Kontextes des Benutzers zu erhalten; und
eine Schaltungsanordnung (124, 224), die mit den Elektroden gekoppelt ist, um einen direkten Kontakt zwischen den Händen, Handflächen oder Handgelenken des Benutzers und den Elektroden zu erkennen und bei Erkennung die ein oder mehreren Parameter des physiologischen Kontextes des Benutzers zu erfassen, während der direkte Kontakt weiterbesteht,
wobei die ein oder mehreren Elektroden ein elektrisch leitendes Muster (160, 220, 302) auf der Arbeitsfläche bilden, **dadurch gekennzeichnet, dass** das elektrisch leitende Muster elektrisch mit einer Sensorfläche einer kapazitiven EKG-Messelektrode (630), die innerhalb der Arbeitsfläche oder auf einer Rückseite der Arbeitsfläche angeordnet ist, gekoppelt ist, wobei die Größe des elektrisch leitenden Musters größer als die Größe der Sensorfläche ist, um die Sensorfläche der kapazitiven Elektrode zu erweitern.

2. Einrichtung nach Anspruch 1, wobei die Schaltungsanordnung umfasst:
wenigstens eine der ein oder mehreren Elektroden zum Erkennen eines direkten Kontakts mit einem bestimmten elektrischen Potenzial; und
einen Komparator, der mit der wenigstens einen Elektrode gekoppelt ist, um eine Änderung in dem bestimmten elektrischen Potenzial zu erkennen, wobei die Änderung durch den direkten Kontakt der wenigstens einen Elektrode mit den Händen, Handflächen oder Handgelenken des Benutzers verursacht wird, wobei der Komparator einen Ausgang bereitstellen soll, die das Einschalten des elektrisch leitenden Musters ermöglicht, als Ergebnis der Erfassung der Änderung in dem bestimmten elektrischen Potenzial, wobei die Schaltungsanordnung ferner ein Frontend-Sensormodul zum Empfangen und Vorverarbeiten von Messwerten umfasst, die durch das elektrisch leitende Muster während des direkten Kontakts mit den Händen, Handflächen oder Handgelenken des Benutzers bereitgestellt werden, wobei der Komparatorausgang ferner das Einschalten des Frontend-Sensormoduls und des elektrisch leitenden Musters ermöglicht.

3. Einrichtung nach Anspruch 2, wobei das elektrisch leitende Muster ein ausgewähltes der folgenden umfasst: ein Kammmuster, ein Zickzackmuster, ein Wellenmuster oder ein Girlandenmuster.

4. Einrichtung nach Anspruch 2, wobei die Arbeitsfläche ein Substrat umfasst, wobei das elektrisch leitende Muster auf einer Außenseite des Substrats angeordnet ist und wobei die kapazitive Elektrode auf einer Innenseite des Substrats angeordnet ist, wobei das elektrisch leitende Muster auf dem Substrat durch Filmabscheidung, Ätzen oder Anbringen eines das Muster umfassenden, elektrisch leitenden Aufklebers am Substrat angeordnet wird.

5. Einrichtung nach Anspruch 2, wobei das elektrisch leitende Muster wenigstens zwei Elektrokardiogramm (EKG) -Elektroden umfasst.

6. Einrichtung nach Anspruch 2, wobei das elektrisch leitende Muster mit einem oder mehreren der folgenden gekoppelt ist: einem Temperatursensor, um die Körpertemperatur des Benutzers bereitzustellen, oder einem optischen Sensor, um ein Photoplethysmogramm (PPG) des Benutzers bereitzustellen, wobei die Schaltungsanordnung dazu dient, die Parameter des physiologischen Kontextes des Benutzers zur weiteren Verarbeitung an eine mit der Einrichtung verbundene Verarbeitungseinheit bereitzustellen, wobei der physiologische Kontext des Benutzers wenigstens einige der folgenden umfasst: elektrokardiographische Daten, photoplethysmographische Daten, Blutdruck, Temperatur und Atmung.

7. Einrichtung nach Anspruch 1, wobei die Einrichtung ein Laptop-Computer oder ein Desktop-Computer ist, wobei die Arbeitsfläche einen Teil einer Tastatur des Laptop-Computers oder des Desktop-Computers umfasst.

8. Einrichtung nach einem der Ansprüche 1 bis 7, wobei die Einrichtung ein Tablet-Computer oder ein Smartphone ist, wobei die Arbeitsfläche eine ausgewählte Fläche aus einer Einfassung des Tablet-Computers oder einer Rückseite des Tablet-Computers oder des Smartphones umfasst.

9. Einrichtung nach Anspruch 1, ein Gehäuse umfassend, das wenigstens eine Arbeitsfläche aufweist.

10. Einrichtung nach Anspruch 9, wobei das Gehäuse wenigstens einen Teil einer Tastatur der Einrichtung, einer Einfassung der Einrichtung oder einer Rückseite der Einrichtung umfasst.

11. Einrichtung nach Anspruch 10, wobei die Einrichtung eines der folgenden umfasst: einen Laptop-Computer, einen Desktop-Computer, einen Tablet-Computer oder ein Smartphone.

12. Verfahren (1000) zum Montieren einer Einrichtung für opportunistische Messungen des Kontextes eines Benutzers, wobei die Messungen EKG-Messungen umfassen, wobei das Verfahren umfasst:
Anordnen (1002) mehrerer Elektroden, die ein elektrisch leitendes Muster umfassen, auf einer Arbeitsfläche einer Computervorrichtung;
Anordnen (1004) einer Schaltungsanordnung in der Computervorrichtung, um einen direkten Kontakt zwischen den Händen oder Handgelenken des Benutzers und dem elektrisch leitenden Muster zu erkennen und einen oder mehrere Parameter des physiologischen Kontextes eines Benutzers während des direkten Kontakts zu erfassen; und
elektrisches Koppeln (1006) der Schaltungsanordnung mit dem elektrisch leitenden Muster,
**dadurch gekennzeichnet, dass**
das elektrisch leitende Muster elektrisch mit einer Sensorfläche einer kapazitiven EKG-Messelektrode gekoppelt ist, die innerhalb der Arbeitsfläche oder auf einer Rückseite der Arbeitsfläche angeordnet ist, wobei die Größe des elektrisch leitenden Musters größer als die Größe der Sensorfläche ist, um die Sensorfläche der kapazitiven Elektrode zu erweitern.

13. Verfahren nach Anspruch 12, wobei das Anordnen eines elektrisch leitfähigen Musters das Ätzen, das Abscheiden des elektrisch leitfähigen Musters auf einem Substrat, das die Arbeitsoberfläche umfasst, oder das Anbringen eines das Muster umfassenden, elektrisch leitfähigen Aufklebers am Substrat umfasst.

14. Verfahren nach Anspruch 12 oder 13, ferner umfassend: kommunikationsfähiges Koppeln der Schaltungsanordnung mit einer Verarbeitungseinheit der Computervorrichtung zum Verarbeiten eines oder mehrerer Parameter des physiologischen Kontextes des Benutzers.

## Revendications

1. Appareil (100, 200, 300) de mesures opportunistes d'un contexte d'utilisateur, lesdites mesures comportant des mesures d'ECG, ledit appareil étant un dispositif informatique comprenant :
au moins une surface de travail (102, 202, 304) qui comporte une ou plusieurs électrodes (110, 112, 114, 116 ; 210, 212, 214, 216) disposées sur la surface de travail pour entrer directement en contact avec les mains, les paumes ou les poignets d'un utilisateur, quand les mains, paumes ou poignets de l'utilisateur sont disposés sur la surface de travail pour interagir avec l'appareil, afin d'obtenir un ou plusieurs paramètres de contexte physiologique de l'utilisateur ; et
des circuits (124, 224) couplés aux électrodes pour détecter un contact direct entre les mains, paumes ou poignets de l'utilisateur et les électrodes et lors de la détection collecter les un ou plusieurs paramètres du contexte physiologique de l'utilisateur pendant que le contact direct est maintenu,
dans lequel les une ou plusieurs électrodes forment un motif électriquement conducteur (160, 220, 302) sur la surface de travail, **caractérisé en ce que** le motif électriquement conducteur est couplé électriquement à une surface de détection d'une électrode de mesure d'ECG capacitive (630) disposée à l'intérieur de la surface de travail ou sur une face arrière de la surface de travail, la taille du motif électriquement conducteur étant supérieure à la taille de la surface de détection afin d'agrandir la surface de détection de l'électrode capacitive .

2. Appareil selon la revendication 1, dans lequel les circuits comprennent :
au moins une des une ou plusieurs électrodes pour détecter un contact direct, présentant un potentiel électrique déterminé ; et
un comparateur couplé à l'au moins une électrode pour détecter un changement du potentiel électrique déterminé, le changement étant causé par le contact direct de l'au moins une électrode avec les mains, paumes ou poignets de l'utilisateur, le comparateur étant destiné à fournir une sortie qui permet la mise sous tension du motif électriquement conducteur à la suite de la détection du changement du potentiel électrique déterminé, les circuits comprenant en outre un module capteur frontal pour recevoir et prétraiter des lectures fournies par le motif électriquement conducteur durant le contact direct avec les mains, paumes ou poignets de l'utilisateur, la sortie du comparateur permettant en outre la mise sous tension du module capteur frontal et du motif électriquement conducteur.

3. Appareil selon la revendication 2, dans lequel le motif électriquement conducteur comprend une forme sélectionnée parmi une forme de peigne, une forme de zigzag, une forme de vague, ou une forme de guirlande.

4. Appareil selon la revendication 2, dans lequel la surface de travail comprend un substrat, le motif électriquement conducteur est disposé sur une face externe du substrat, l'électrode capacitive est disposée sur une face interne du substrat, et le motif électriquement conducteur est disposé sur le substrat par dépôt de film, gravure ou fixation d'un autocollant électriquement conducteur comprenant le motif sur le substrat.

5. Appareil selon la revendication 2, dans lequel le motif électriquement conducteur comprend au moins deux électrodes d'électrocardiogramme (ECG).

6. Appareil selon la revendication 2, dans lequel le motif électriquement conducteur est couplé à un ou plusieurs : d'un capteur de température pour fournir la température corporelle de l'utilisateur, d'un capteur optique pour fournir un photopléthysmogramme (PPG) de l'utilisateur, les circuits étant destinés à fournir les paramètres du contexte physiologique de l'utilisateur à une unité de traitement associée à l'appareil en vue de leur traitement ultérieur, le contexte physiologique de l'utilisateur comprenant au moins certaines de : données électrocardiographiques, données photopléthysmographiques, pression artérielle, température et respiration.

7. Appareil selon la revendication 1, l'appareil étant un ordinateur portable ou un ordinateur de bureau, la surface de travail comprenant une partie d'un clavier de l'ordinateur portable ou de l'ordinateur de bureau.

8. Appareil selon l'une quelconque des revendications 1 à 7, l'appareil étant une tablette informatique ou un téléphone intelligent, la surface de travail comprenant une face avant ou arrière sélectionnée de la tablette informatique ou du téléphone intelligent.

9. Appareil selon la revendication 1, comprenant un boîtier, présentant l'au moins une surface de travail.

10. Appareil selon la revendication 9, dans lequel le boîtier comprend au moins une partie d'un clavier de l'appareil, une face avant de l'appareil ou une face arrière de l'appareil.

11. Appareil selon la revendication 10, l'appareil comprenant au moins un : d'un ordinateur portable, d'un ordinateur de bureau, d'une tablette informatique ou d'un téléphone intelligent.

12. Procédé (1000) d'assemblage d'un appareil de mesures opportunistes d'un contexte d'utilisateur, lesdites mesures comportant des mesures d'ECG, et le procédé comprenant :
la disposition (1002) d'une pluralité d'électrodes comprenant un motif électriquement conducteur sur une surface de travail d'un dispositif informatique ;
la disposition (1004) de circuits dans le dispositif informatique, pour détecter un contact direct entre les mains ou poignets de l'utilisateur et le motif électriquement conducteur et collecter un ou plusieurs paramètres d'un contexte physiologique de l'utilisateur durant le contact direct ; et
le couplage électrique (1006) des circuits avec le motif électriquement conducteur,
**caractérisé en ce que**
le motif électriquement conducteur est couplé électriquement à une surface de détection d'une électrode de mesure d'ECG capacitive disposée à l'intérieur de la surface de travail ou sur une face arrière de la surface de travail, la taille du motif électriquement conducteur étant supérieure à la taille de la surface de détection afin d'agrandir la surface de détection de l'électrode capacitive.

13. Procédé selon la revendication 12, dans lequel la disposition d'un motif électriquement conducteur comprend la gravure, le dépôt du motif électriquement conducteur sur un substrat comprenant la surface de travail, ou la fixation d'un autocollant électriquement conducteur comprenant le motif sur le substrat.

14. Procédé selon la revendication 12 ou 13, comprenant en outre : le couplage de manière communicante des circuits avec une unité de traitement du dispositif informatique, en vue du traitement des un ou plusieurs paramètres du contexte physiologique de l'utilisateur.
